# EUROPÄISCHE PATENTANMELDUNG

(11) **EP 2 883 551 A1**
(43) Veröffentlichungstag der Anmeldung: **17.06.2015**
(21) Anmeldenummer: 14197873.4
(22) Anmeldetag: 15.12.2014
(51) Int. Cl.: A61L 2/00, A61M 39/10, A61L 2/04

(54) **Verfahren und Vorrichtung zur Erwärmung von Volumina von Medien in einem geschlossenen Behälter mittels einer elektromagnetischen Strahlung**

(30) Priorität: 16.12.2013 DE 102013114101
(71) Anmelder: Askion GmbH, 07549 Gera (DE); Gerald Wagner Consulting LLC, Upper Grandview, NY 10960 (US)
(72) Erfinder: Doms, Lutz, 08543 Pöhl (DE); Wagner, Gerald, Upper Grandview, NY 10960 (US)
(74) Vertreter: Oehmke, Volker

(57) **Zusammenfassung**

Die Erfindung betrifft ein Verfahren zur Erwärmung von Volumina von Medien (11) in einem geschlossenen Behälter (10) mittels einer elektromagnetischen Strahlung (2), bei dem anhand von Messungen an Referenzbehältern eine Datenbank (4) erstellt wird und unter Nutzung der Datenbank (4) Bestrahlungsregime ausgewählt oder erzeugt werden, die für die Erwärmung des Mediums (11) verwendet werden. Durch das erfindungsgemäße Verfahren sind von einem Anwender des Verfahrens keine Kenntnisse der thermodynamischen und materialtechnischen Eigenschaften des Behälters (10) gefordert. Die Erfindung betrifft außerdem eine Vorrichtung zur Durchführung des Verfahrens.

## Beschreibung

Die Erfindung betrifft ein Verfahren und eine Vorrichtung zur Erwärmung von Volumina von Medien in einem geschlossenen Behälter mittels einer elektromagnetischen Strahlung.

In medizinischen Anwendungen und Therapieverfahren ist es häufig erforderlich, einem Patienten einen ständigen Zugang zu legen. Über diesen Zugang können dann bei Bedarf Lösungen mit Medikamenten oder sonstigen Inhaltsstoffen dem Patienten zugeführt werden.

Eine typische Anwendung ist bei Dialysepatienten gegeben, die öfters an ein Dialysegerät angeschlossen werden müssen. Dazu ist es möglich, dass an dem ständigen Zugang, der an dem Patienten verbleibt, eine Kupplungsvorrichtung vorhanden ist, über die der Zugang mit einem Dialysegerät verbunden werden kann.

Dabei ist es natürlich außerordentlich wichtig, dass bei einem erneuten Anschluss der Kupplungsvorrichtung eine Infektionsgefahr für den Patienten so weit als möglich ausgeschlossen wird.

In einer Veröffentlichung von Fessia et al. (1990, Moist heat intraluminal disinfection of CAPD connectors, Perit.Dial.Bull. 6: 164 - 168) ist ein Verfahren zur Desinfektion solcher Kupplungsvorrichtungen beschrieben. Üblicherweise bestehen die Kupplungsvorrichtungen aus zwei Teilen, deren Verbindungsbereiche zueinander korrespondieren. Dieses System wird auch als Luer®-Lock oder Luer®-Verbinder bezeichnet (ISO 594-1: 1986; EN 20594-1:1993; DIN EN 20594-1:1996). Die Kupplungsvorrichtung weist im geschlossenen Zustand einen Innenraum auf, durch den beispielsweise die Dialyselösung fließt. Um vor einer Behandlung die Kupplungsvorrichtung zu desinfizieren, wird diese mit der Dialyselösung (fortan: Medium) gefüllt und die Zu- und Ableitung der Dialyselösung verschlossen. Anschließend wird die Kupplungsvorrichtung so lange mit Mikrowellenstrahlung bestrahlt, bis das Medium eine Temperatur von mindestens 100°C aufweist. Die von Fessia et al. angeführte Bestrahlungsdauer beträgt 54 Sekunden. Mittels dieses Verfahrens konnten die 106 der getesteten Mikroorganismen (gram-positive und gramnegative Bakterien, Hefen und Pilze) soweit reduziert werden, dass die Erfordernisse einer Desinfektion (Keimzahlreduktion um mindestens den Faktor 10⁵) erfüllt waren.

Grabowy et al. (1998, New connection method for isolating and disinfecting intraluminal path during peritoneal dialysis solution-exchange procedures, Adv.Perit.Dial. 14:149-153) beschreiben eine Kupplungsvorrichtung, für ein Dialysegerät, deren Zu- und Ableitungen druckdicht verschlossen wurden und das Medium mit Mikrowellenstrahlung erwärmt wird. Innerhalb von etwa 10 Sekunden wird das Medium von 25°C auf Temperaturen über 100°C erwärmt, wasist durch den druckdichten Verschluss ermöglicht ist. Durch den Verschluss ist zudem erreicht, dass keine möglicherweise kontaminierten Medienanteile austreten können und eben das gesamte möglicherweise kontaminierte Medium der Erwärmung unterzogen wird. Die Kupplungsvorrichtung, die als Behälter aufgefasst werden kann, besteht aus Kunststoff, der für Mikrowellenstrahlung weitgehend durchlässig (transmissiv) ist.

Mittels der oben beschriebenen Verfahren können Geräte betrieben werden, mit denen die Behälter erwärmt und desinfiziert werden können. Dies ist dann vergleichsweise unproblematisch, wenn die Form, Abmaße, Material etc. der Behälter und der Medien bekannt sind. Allerdings kann die Zusammensetzung von Kunststoffen zwischen verschiedenen Chargen oder von Anbieter zu Anbieter variieren. Auch können die Behälter verschiedener Anbieter aus unterschiedlichen Materialien bestehen. Um aber eine sichere Erwärmung des gesamten Mediums zu garantieren, müssen die materialseitigen Einflüsse auf den Erwärmungsvorgang sowie ggf. unterschiedliche Gestaltungen der Behälter mit berücksichtigt werden.

So besteht nun beispielsweise für medizinisches Personal das erhebliche Problem zu wissen oder in Erfahrung bringen zu müssen, aus welchem Material der gerade zu verwendende Behälter besteht.

Der Erfindung liegt die Aufgabe zugrunde, ein verbessertes Verfahren zur Erwärmung von Volumina von Medien in einem geschlossenen Behälter mittels einer elektromagnetischen Strahlung vorzuschlagen, die für eine Vielzahl von Behältern unterschiedlicher thermodynamischer Eigenschaften einsetzbar ist. Der Erfindung liegt außerdem die Aufgabe zugrunde, eine Vorrichtung zur Durchführung des Verfahrens vorzuschlagen.

Die Aufgabe wird in einem Verfahren zur Erwärmung von Volumina von Medien in einem geschlossenen Behälter mittels einer elektromagnetischen Strahlung gelöst, wobei
vor der Durchführung der Erwärmung eine Datenbank angelegt wird, indem
- in einem Schritt A: mindestens ein Referenzbehälter aus einem bekannten Material, der ein bekanntes Volumen aufweist und ein bekanntes Medium enthält, mit jeweils einer ersten bis n-ten Strahlung über je eine Zeitdauer bestrahlt wird, wobei die Eigenschaften der ersten bis n-ten Strahlung bekannt sind,
- in einem Schritt B: über die Zeitdauer der Bestrahlung zu bekannten Messzeitpunkten eine Anzahl Messwerte mindestens einer Messgröße erhoben und als dem Referenzbehälter zugeordnete Messreihen erfasst und abrufbar als Referenzmessreihen abgespeichert werden,
- in einem Schritt C: aus den Referenzmessreihen diejenigen Referenzmessreihen ausgewählt werden, bei deren Erstellung innerhalb einer ausgewählten Bestrahlungsdauer eine ausgewählte Energiemenge in den Referenzbehälter eingekoppelt worden ist,
- in einem Schritt D: von den ausgewählten Referenzmessreihen jeweils ein Bestrahlungsregime abgeleitet wird und diesen ausgewählten Referenzmessreihen zugeordnet abgespeichert wird,
und bei dem bei der Durchführung der Erwärmung
- in einem Schritt a: der Behälter mit der Strahlung, deren Eigenschaften bekannt sind, über eine bekannte Bestrahlungsdauer bestrahlt wird und mindestens ein dem Behälter und einem Messzeitpunkt innerhalb der Bestrahlungsdauer zugeordneter Messwert einer Messgröße erhoben und auf das Vorliegen einer Übereinstimmung mit den Werten von in einer Datenbank abgelegten Referenzmessreihen überprüft wird,
- in einem Schritt b: danach bei einer Übereinstimmung des mindestens einen Messwertes mit einem Wert einer Referenzmessreihe ein der betreffenden Referenzmessreihe zugeordnetes Bestrahlungsregime ausgewählt wird, und
- in einem Schritt c: danach die Erwärmung des Behälters unter Anwendung des ausgewählten Bestrahlungsregimes bis zu einem vorbestimmten Temperaturendpunkt fortgesetzt wird.

Durch die Erfindung ist eine Möglichkeit vorgeschlagen, mit der eine Erwärmung des Mediums in dem Behälter durchgeführt werden kann, ohne dass ein Anwender des Verfahrens genaue Kenntnisse von den Eigenschaften des Behälters benötigt. So kann beispielsweise der Behälter ein Luer®-Verbinder (oder auch Luer®-Lock) sein, in dem sich ein Medium befindet, dessen möglicherweise enthaltene Keimzahl durch Erwärmung reduziert werden soll.

Die Erwärmung des Mediums erfolgt nicht unter Durchflussbedingungen. Ein Volumen des Mediums ist für die Dauer der Durchführung der Schritte A bis D bzw. der Schritte a bis c in einem Innenraum des Behälters bzw. des Referenzbehälters vorzugsweise blasenfrei eingeschlossen.

Es ist in der Regel ausreichend, wenn die Schritte A bis D einmalig vor einer ersten Verwendung des erfindungsgemäßen Verfahrens durchgeführt werden. Die Schritte a bis c können beliebig oft wiederholt werden. Während der Durchführung der Schritte a bis c können die in den Schritten B bis D erhobenen Messwerte, Referenzmessreihen und Bestrahlungsregime ergänzt und / oder korrigiert werden sowie neue Messwerte, Referenzmessreihen und Bestrahlungsregime der Datenbank hinzugefügt werden.

Unter dem Begriff der Messgrößen werden physikalische Größen wie z. B. Temperatur, Zeit, Größe der elektromagnetischen Strahlung, Phasenlage der elektromagnetischen Strahlung und Frequenz der elektromagnetischen Strahlung verstanden. Es hat sich als günstig erwiesen, wenn von den Messgrößen eine oder mehrere ausgewählt werden und von diesen ausgewählten Messgrößen dann abgeleitete Messgrößen gebildet werden, die als Messwerte der Referenzmessreihen in Schritt a und als Messwerte in Schritt B verwendet werden. Besonders günstig ist es, wenn als abgeleitete Messgröße der Realteil und / oder der Imaginärteil der Permittivität verwendet wird.

Eine erste elektromagnetische Strahlung (vereinfachend auch: erste Strahlung) ist eine zur Erwärmung des Mediums geeignete Strahlung, z. B. Mikrowellenstrahlung, mit bekannten Eigenschaften, beispielsweise einer ersten Wellenlänge, einer ersten Phasenlage oder einer ersten Leistung. Jede der ersten bis n-ten Strahlung unterscheidet sich in mindestens einer Eigenschaft von den anderen Strahlungen.

Eine Referenzmessreihe wird aufgrund der in Schritt B ermittelten Messwerte erstellt. Dabei kann aus der Referenzmessreihe eine Funktion abgeleitet werden, durch die die Referenzmessreihe approximiert ist. Eine solche Approximation kann mittels dem Fachmann bekannter Regressionsverfahren erfolgen.

Es ist in einer vorteilhaften Ausgestaltung des erfindungsgemäßen Verfahrens möglich, dass zur Erstellung der Referenzmessreihen durch einen Sensor die Temperatur des Mediums erfasst und einer erfassten Temperatur der Außenwand des Referenzbehälters zugeordnet gespeichert wird. Es wird so eine Korrelation zwischen den Temperaturen der Außenwand und dem Medium erhalten. Damit ist es vorteilhaft ermöglicht, während der Verfahrensdurchführung allein aus der Temperatur der Außenwand auf die Temperatur des Mediums zu schließen.

Beispielsweise ist eine solche Funktion durch eine Regressionskurve einer Anzahl von Messwerten zu verschiedenen Messzeitpunkten und / oder an verschiedenen Orten, erhalten. Für eine solche Regressionskurve können zudem mittels verschiedener bekannter Verfahren verschiedene Vertrauensbereiche (Konfidenzbereiche, confidence limits), beispielsweise 90, 95 oder 99%-Vertrauensbereiche, berechnet werden, wie dies dem Fachmann ganz allgemein aus dem Gebiet der deskriptiven Statistik und der Messtechnik bekannt ist. Bei einem 95%-Vertrauensbereich liegen beispielsweise 95% aller aus den Funktionswerten berechenbaren Vertrauensbereiche innerhalb des 95%-Vertrauensbereiches.

Die Vertrauensbereiche im Sinne dieser Beschreibung können in weiteren Ausführungen des erfindungsgemäßen Verfahrens auch willkürlich festgelegt sein. Sie können ferner aufgrund einer Vorschrift geschätzt oder berechnet werden, die von der oben genannten Definition eines Vertrauensbereiches abweichen.

Nachfolgend wird angenommen, dass für die Durchführung einer bestimmten Ausgestaltung des erfindungsgemäßen Verfahrens jeweils eine konkrete Definition des Vertrauensbereiches festgelegt ist.

Ein in dem Schritt a erhobener Messwert stimmt vorzugsweise dann mit einer Referenzmessreihe überein, wenn dieser innerhalb des Vertrauensbereiches dieser Referenzmessreihe liegt.

Von der wie vorstehend ermittelten Funktion kann ein Bestrahlungsregime abgeleitet werden. Dabei ist das abgeleitete Bestrahlungsregime im einfachsten Fall durch eine Bestrahlungsdauer und eine Strahlung gegeben, aufgrund der die betreffende Referenzmessreihe, und nachfolgend deren Funktion erhalten wurde. Es ist auch möglich, dass ein Bestrahlungsregime zwar von einer Referenzmessreihe oder deren Regressionskurve bzw. Funktion abgeleitet wird, aber nicht oder nicht vollständig mit dem Bestrahlungsregime (Bestrahlungsdauer und Strahlung) übereinstimmt, welches zu der Referenzmessreihe geführt hat.

Ein Bestrahlungsregime gibt an, mit welchen Parametern, z. B. abgestrahlte Leistung der elektromagnetischen Strahlung, Zeitdauer der Bestrahlung (Bestrahlungsdauer) des Referenzbehälters bzw. des Behälters und ggf. Veränderungen von Eigenschaften der elektromagnetischen Strahlung, wie abgestrahlte Leistung, Wellenlänge und / oder Phasenlage über die Bestrahlungsdauer, eine Bestrahlung und damit eine Erwärmung des Behälters erfolgt. Ziel ist es, mittels eines gewählten Bestrahlungsregimes eine vorbestimmte Energiemenge in das Medium einzubringen und die Temperatur des Mediums zuverlässig und überall auf eine gewünschte Temperatur (Solltemperatur, Temperaturendpunkt) zu bringen. Möglicherweise erforderliche Anlaufzeiten, beispielsweise zwischen einer Ansteuerung einer Strahlungsquelle und der Abstrahlung der elektromagnetischen Strahlung, sind vorzugsweise in dem Bestrahlungsregime berücksichtigt.

Ein Bestrahlungsregime kann beispielsweise durch ein Diagramm veranschaulicht werden, das als Funktionsbereich die Zeit und als Wertebereiche zum einen die Temperatur des Mediums und zum anderen Werte einer Eigenschaft der Strahlung (z. B. Leistung, Frequenz, Wellenlänge) aufweist. Die über der Zeit aufgetragenen Messwerte der Temperaturen geben die Referenzmessreihe an. Durch die Eigenschaft der Strahlung über der Zeit ist das Bestrahlungsregime angegeben, welches zu der dargestellten Referenzmessreihe geführt hat. Eine solche Darstellung kann natürlich mehrdimensional erfolgen, wenn sich beispielsweise mehrere Eigenschaften der Strahlung über die Zeit ändern.

Ein Bestrahlungsregime kann ferner von einer bekannten Funktion, beispielsweise einer Regression einer Referenzmessreihe, abgeleitet werden, die inter- und / oder extrapoliert wurde. Es ist auch möglich, dass eine anhand von Messwerten des Schrittes B ermittelte oder geschätzte Funktion einer Referenzmessreihe transformiert, beispielsweise linearisiert wird. Für transformierte Funktionen können beispielsweise Vertrauensbereiche in Form von zulässigen Klassen oder Klassenbreiten definiert werden.

Unter dem Begriff der Reflektivität wird nachfolgend der an einer Oberfläche eines Körpers, beispielsweise eines Messkörpers, reflektierte Anteil einer auf den Körper gerichteten elektromagnetischen Strahlung (nachfolgend auch kurz: Strahlung) verstanden.

Entsprechend bezeichnet der Begriff der Transmissivität den Anteil der elektromagnetischen Strahlung, der durch den Körper bzw. den Messkörper hindurch dringt (transmittierte Strahlung). Die elektromagnetische Strahlung kann bei dem Durchdringen des Körpers verändert werden. So kann beispielsweise die Phasenlage sowie die Frequenz bzw. das Frequenzband der transmittierten Strahlung verändert sein.

Durch die Permittivität ist die Durchlässigkeit eines Materials für elektromagnetische Felder beschrieben. Die Permittivität ist temperatur- und / oder frequenzabhängig. Dielektrische Verluste der elektromagnetischen Strahlung in dem Material können durch den Imaginärteil der Permittivität angegeben werden, während durch den Realteil das Vermögen des Materials, ein elektromagnetisches Feld durchzulassen, beschrieben ist. Weist die Permittivität sowohl Real- als auch Imaginärteil auf, kann die frequenzabhängige Phasenverschiebung ermittelt werden.

Bei einem Durchgang der elektromagnetischen Strahlung durch ein Medium, wie z. B. einen Körper, eine Flüssigkeit, eine Suspension oder eine Dispersion, kann deren Phasenlage frequenzabhängig verschoben werden. Eine solche frequenzabhängige Phasenverschiebung tritt dann auf, wenn durch das Material auch dielektrische Verluste verursacht sind, so dass dessen Permittivität durch einen Realteil und einen Imaginärteil zu beschreiben ist.

Der Temperaturendpunkt ist durch eine Temperatur gegeben, bei deren Erreichen das Medium eine gewünschte Temperatur aufweist, und zwar überall. Es sollen also keine Bereiche in dem Medium vorhanden sein, an denen die gewünschte Temperatur des Mediums nicht erreicht wird. Außerdem soll diese gewünschte Temperatur über eine hinreichend lange Zeitdauer von beispielsweise 5, 10, 15, 20, 25, 30 Sekunden oder mehr als 30 Sekunden beibehalten werden.

Der Referenzbehälter besteht aus einem bekannten Material, welches vorzugsweise hinsichtlich seines Erwärmungsverhaltens bei Bestrahlung mit der elektromagnetischen Strahlung gleiche Eigenschaften aufweist, wie ein Material, aus dem erwartungsgemäß der Behälter gefertigt sein wird. Ebenso sind das Volumen und die Form eines Innenraums des Referenzbehälters bekannt und gleich oder ähnlich dem erwarteten Volumen und der Form eines Innenraums eines Behälters. Vorzugsweise sind auch Außenabmaße und Wandstärken des Referenzbehälters bekannt und gleich oder ähnlich den entsprechenden Maßen des Behälters. Das bekannte Medium in dem Referenzbehälter und das zu erwärmende Medium in dem Behälter sind vorzugsweise gleich. Sollte die Verwendung eines gleichen Mediums nicht möglich sein, so sollen die Medien einander, insbesondere hinsichtlich ihres Erwärmungsverhaltens und des Absorptionsverhaltens der ersten bis n-ten Strahlungen, so ähnlich wie möglich sein.

Während der Durchführung des erfindungsgemäßen Verfahrens ist es möglich, dass während der Erwärmung weitere Messwerte erhoben werden und eine Übereinstimmung mindestens einer Auswahl der weiteren Messwerte mit Werten derjenigen Referenzmessreihe (fortan auch: ausgewählte Referenzmessreihe), der das ausgewählte Bestrahlungsregime zugeordnet ist, überprüft wird. Eine Übereinstimmung kann auch dann vorliegen, wenn einzelne weitere Messwerte nicht in dem Vertrauensbereich liegen, aber eine Regressionskurve der weiteren Messwerte innerhalb des Vertrauensbereiches liegt.

Durch eine solche Gestaltung des erfindungsgemäßen Verfahrens ist vorteilhaft die Möglichkeit eröffnet, zu kontrollieren, ob die Auswahl des Bestrahlungsregimes tatsächlich zutreffend ist. Erfolgte beispielsweise die Auswahl eines Bestrahlungsregimes anhand eines einzigen Messwertes oder anhand von Messwerten, die innerhalb einer kurzen Zeitdauer ermittelt wurden, kann anhand weiterer Messwerte überprüft werden, ob dieselbe Auswahl auch mit den weiteren Messwerten getroffen würde.

Wird eine fehlende Übereinstimmung festgestellt, so wird in einer weiteren Ausgestaltung des erfindungsgemäßen Verfahrens ein anderes Bestrahlungsregime ausgewählt und die Erwärmung mit dem ausgewählten anderen Bestrahlungsregime fortgesetzt. Diese Verfahrensführung ist insbesondere von Bedeutung, wenn anhand weiterer Messwerte festgestellt wird, dass diese weiteren Messwerte nicht im Vertrauensbereich der Referenzmessreihe liegen. Liegen die weiteren Messwerte beispielsweise im Vertrauensbereich einer anderen Referenzmessreihe, so kann das Bestrahlungsregime, das dieser anderen Referenzmessreihe zugeordnet ist, ausgewählt und weiter verwendet werden.

Es ist eine mögliche Ausgestaltung des Verfahrens, dass ein Bestrahlungsregime erstellt und abgespeichert wird, welches aus den beiden vorgenannten Bestrahlungsregimen zusammengesetzt wird. Alternativ kann anhand der beiden beteiligten Referenzmessreihen ein neues Bestrahlungsregime festgelegt werden. Sind die weiteren Messwerte den Vertrauensbereichen mehrerer Referenzmessreihen zugeordnet, gilt Vorstehendes entsprechend.

Es ist auch möglich, dass bei einer festgestellten fehlenden Übereinstimmung der weiteren Messwerte mit dem Vertrauensbereich der zuerst verwendeten Referenzmessreihe die Erwärmung beendet und ein Fehlersignal generiert wird. Das Fehlersignal kann auch generiert werden, wenn kein anderes Bestrahlungsregime auswählbar ist. Das Fehlersignal kann optisch und / oder akustisch sein. Die Generierung eines Fehlersignals kann in einer weiterführenden Ausgestaltung des Verfahrens protokolliert werden.

Ferner kann das erfindungsgemäße Verfahren derart ausgestaltet sein, dass bei einer fehlenden Übereinstimmung mindestens eines weiteren Messwertes aufgrund der bis dahin erfassten weiteren Messwerte ein prognostiziertes Bestrahlungsregime berechnet wird und die Erwärmung mit dem prognostizierten Bestrahlungsregime fortgesetzt wird. Bei einer solchen Verfahrensgestaltung ist das prognostizierte Bestrahlungsregime nicht an das Vorhandensein einer Referenzmessreihe gebunden. Es können aber aus der Datenbank und den dort abgelegten Referenzmessreihen, Vertrauensbereichen und Bestrahlungsregimen Regeln für die Berechnung des prognostizierten Bestrahlungsregimes abgeleitet werden.

Das prognostizierte Bestrahlungsregime kann in die Datenbank übernommen und wiederholt abrufbar gespeichert werden. Das prognostizierte Bestrahlungsregime kann für weitere Berechnungen prognostizierter Bestrahlungsregime berücksichtigt werden (selbstlernende Datenbank).

Eine Messgröße kann aus einer Reflektivität des Behälters, einer Transmissivität des Behälters, mindestens einer Temperatur an mindestens einem ausgewählten Punkt oder Bereich des Behälters oder einer frequenzabhängigen Phasenverschiebung eines den Behälter durchdringenden Anteils der Strahlung ausgewählt werden.

Es können in weiteren Ausgestaltungen des Verfahrens auch mindestens zwei Messgrößen aus einer Gruppe von Messgrößen, bestehend aus einer Reflektivität, einer Transmissivität, einer Temperatur und einer Phasenverschiebung der Strahlung, ausgewählt werden.

Das erfindungsgemäße Verfahren kann äußerst vorteilhaft zur Desinfektion des Inneren des Behälters verwendet werden. Dabei ist der Behälter vorzugsweise ein medizinisches Gerät oder ein Bestandteil eines medizinischen Geräts. In einer bevorzugten Verwendung des Verfahrens ist der Behälter ein Luer®-Verbinder (Luer®-Lock).

Die Aufgabe wird ferner durch eine Vorrichtung zur Erwärmung der Volumina von Medien in einem geschlossenen Behälter mittels einer elektromagnetischen Strahlung gelöst. Die Vorrichtung weist eine Strahlungsquelle zur Bereitstellung einer elektromagnetischen Strahlung, einen Messraum zur Aufnahme des Behälters und eine Abstrahleinrichtung zur gerichteten Abstrahlung der Strahlung in den Messraum, sowie eine Regel- und Steuereinheit zur Regelung und Steuerung der Bereitstellung und der Abstrahlung der Strahlung entsprechend eines Bestrahlungsregimes und ist dadurch gekennzeichnet, dass die Regel- und Steuereinheit mit mindestens einem Sensor in Verbindung steht und der mindestens eine Sensor zur Erfassung mindestens eines dem Behälter zugeordneten Messwerts einer Messgröße ausgebildet ist, die Regel- und Steuereinheit datentechnisch mit einer Speichereinheit verbunden ist, in der eine Anzahl von Referenzmessreihen sowie eine Anzahl von den jeweiligen Referenzmessreihen zugeordneten Bestrahlungsregimen als eine Datenbank abgelegt sind, und die Regel- und Steuereinheit eine Überprüfungseinheit zur Überprüfung einer Übereinstimmung des mindestens einen erfassten Messwerts mit einem Wert der Referenzmessreihen und, bei festgestellter Übereinstimmung, zur Zuordnung eines Bestrahlungsregimes zu dem erfassten und überprüften Messwert aufweist.

Um ein unbeabsichtigtes Austreten der Strahlung aus dem Messraum zu verhindern, ist dieser vorzugsweise geschlossen ausgebildet. Er kann mehrteilig, z. B. aufklappbar ausgelegt sein, wobei er während der Durchführung der Erwärmung vorzugsweise geschlossen ist.

Die Regel- und Steuereinheit der erfindungsgemäßen Vorrichtung weist vorzugsweise eine Extrapolationseinheit zur Ableitung von Bestrahlungsregimen aus einer Anzahl von Messwerten auf.

Der Sensor ist günstigerweise ein Sensor zur Erfassung mindestens einer Reflektivität des Behälters (Reflektivitätssensor). Ein solcher Sensor kann beispielsweise ein Strahlungssensor wie eine Photodiode, ein CCD-Sensor, ein Photomultiplier oder eine Photozelle sein.

Der Sensor kann zudem ein Sensor zur Erfassung mindestens einer frequenzabhängigen Phasenverschiebung eines den Behälter durchdringenden Anteils der Strahlung sein. Ferner kann der Sensor ein Sensor zur Erfassung einer Transmissivität der elektromagnetischen Strahlung sein. Durch einen entsprechend ausgebildeten Sensor können auch die frequenzabhängige Phasenverschiebung und die Transmissivität gemeinsam erfasst werden.

Der Sensor zur Erfassung mindestens einer frequenzabhängigen Phasenverschiebung kann zusätzlich zu dem Strahlungssensor vorhanden sein (Permittivitätssensor). Die Funktionen des Strahlungssensors und des Sensors zur Erfassung mindestens einer frequenzabhängigen Phasenverschiebung können auch von einem Sensor erfüllt werden.

In einer weiteren Ausgestaltung der erfindungsgemäßen Vorrichtung ist der Sensor ein Sensor zur Erfassung einer Temperatur des Behälters (Temperatursensor). Ein solcher Sensor kann als Messfühler direkt in einem Innenraum des Behälters angeordnet sein. Er kann direkt mit dem Medium in Kontakt stehen. Der Sensor kann auch mit der Oberfläche des Behälters in Verbindung stehen und dadurch eine Erfassung einer Oberflächentemperatur des Behälters ermöglichen.

Der Sensor kann in einer weiteren Ausführung zur Erfassung einer Transmissivität ausgebildet sein (Transmissivitätssensor).

Die verschiedenen Ausführungen und Anordnungen der Sensoren können miteinander kombiniert sein.

Die Strahlungsquelle weist vorzugsweise eine Abstrahleinrichtung zur Abstrahlung der Strahlung auf. Die Abstrahleinrichtung ist vorzugsweise mindestens eine Antenne, kann aber beispielsweise auch mindestens ein Hohlleiter oder mindestens eine andere Einkoppelstruktur sein. Die Form, Dimensionierung und relative Anordnung der Abstrahleinrichtung ist auf das oder die anzuwendenden Bestrahlungsregime abgestimmt.

Die erfindungsgemäße Vorrichtung weist vorzugsweise einen Rahmen auf, durch den die Elemente der Vorrichtung gehalten und relativ zueinander positioniert sind. Durch den Rahmen kann zusätzlich eine Abschirmung der elektromagnetischen Strahlung erreicht sein, um eine Umgebung der Vorrichtung keiner unnötigen Belastung durch die elektromagnetische Strahlung auszusetzen.

Die erfindungsgemäße Vorrichtung kann als ein stationäres Gerät gestaltet sein oder ein Bestandteil eines stationären Gerätes sein. Bevorzugt ist jedoch, die erfindungsgemäße Vorrichtung in einem Gerät zu verwenden, welches durch einen Benutzer einfach an den Einsatzort gebracht werden kann. Vorzugsweise ist die Vorrichtung in einem sogenannten "hand-held"-Gerät angeordnet und kann durch einen Benutzer zu dem Behälter mit dem zu erwärmenden Medium gebracht und dort zur Erwärmung des Mediums verwendet werden.

Das erfindungsgemäße Verfahren und die erfindungsgemäße Vorrichtung werden vorzugsweise so gestaltet und ausgeführt, dass die Anforderungen internationaler Standards, insbesondere des Standards des "The International Pharmacopoeia", erfüllt sind. Sind entsprechende nationale oder regionale Anforderungen höher, so werden vorzugsweise diese durch die Wahl der Verfahrensführung und Verfahrensausgestaltung bzw. durch die Wahl der Ausgestaltung der Vorrichtung eingehalten.

Die Erfindung wird nachfolgend anhand von Ausführungsbeispielen und Abbildungen näher erläutert. Es zeigen:
- Fig. 1: einen schematischen Aufbau eines ersten Ausführungsbeispiels einer erfindungsgemäßen Vorrichtung,
- Fig. 2: einen schematischen Aufbau eines zweiten Ausführungsbeispiels einer erfindungsgemäßen Vorrichtung,
- Fig. 3: einen schematischen Aufbau eines dritten Ausführungsbeispiels einer erfindungsgemäßen Vorrichtung,
- Fig. 4: eine schematisch gezeigte Referenzmessreihe mit einem Vertrauensbereich als Darstellung in einem Diagramm mit der Zeit auf der Abszisse sowie der Temperatur eines zu erwärmenden Mediums auf der Ordinate,
- Fig. 5: ein schematisch gezeigtes Bestrahlungsregime und eine Referenzmessreihe als Darstellung in einem Diagramm mit der Zeit auf der Abszisse sowie der Leistung der Strahlung und der Temperatur eines zu erwärmenden Mediums als jeweilige Ordinaten,
- Fig. 6: eine schematische Darstellung von Referenzmessreihen abgeleiteter Messgrößen, zugeordneter Bestrahlungsregime und von Vertrauensbereichen und
- Fig. 7: eine schematische Darstellung von Messwerten abgeleiteter Messgrößen und extrapolierter Bestrahlungsregime.

Gleiche Bezugszeichen bezeichnen in allen Figuren die gleichen technischen Elemente.

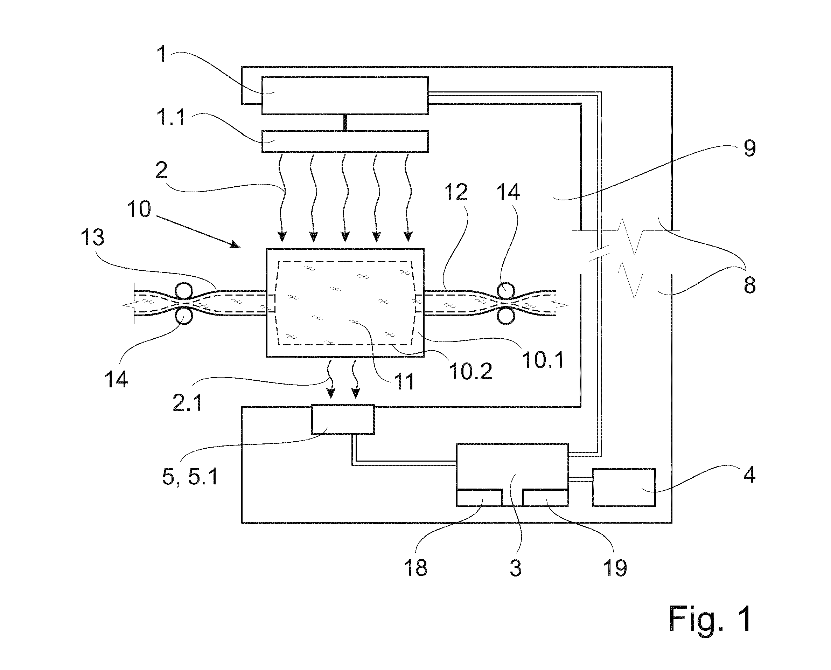

Eine erfindungsgemäße Vorrichtung beinhaltet als wesentliche Elemente eine Strahlungsquelle 1 zur Erzeugung und Bereitstellung einer elektromagnetischen Strahlung 2, eine Abstrahleinrichtung 1.1 zur Abstrahlung der elektromagnetischen Strahlung 2, mindestens einen Sensor 5, eine Regel- und Steuereinheit 3 und eine Datenbank 4 (Fig. 1).

In einem ersten Ausführungsbeispiel einer erfindungsgemäßen Vorrichtung ist, wie in Fig. 1 gezeigt, ein Rahmen 8 vorhanden, an dem die Strahlungsquelle 1, die Abstrahleinrichtung 1.1 und ein zur Erfassung einer Permittivität der elektromagnetischen Strahlung 2 ausgebildeter Sensor 5 (fortan: Permittivitätssensor 5.1) gehalten und in einer vorbestimmten Weise zueinander positioniert sind. Der Rahmen 8 ist Teil eines Gehäuses eines Gerätes (nicht gezeigt). In weiteren Ausgestaltungen der Vorrichtung kann der Rahmen 8 auch unabhängig von einem Gehäuse oder einem Gerät gestaltet sein. Durch einen Teil des Rahmens 8 ist ein Messraum 9 umfangen, in dem ein Behälter 10, der auch ein Referenzbehälter sein kann, zur Durchführung der Erwärmung eines Mediums 11 in dem Behälter 10 angeordnet werden kann. Die Strahlungsquelle 1 steht mit der Abstrahleinrichtung 1.1 in Verbindung, mittels der die von der Strahlungsquelle 1 bereitgestellte elektromagnetische Strahlung 2 von einer Seite des Messraums 9 gerichtet in den Messraum 9 abstrahlbar ist. Gegenüber der Abstrahleinrichtung 1.1 ist auf der anderen Seite des Messraums 9 der Permittivitätssensor 5.1 so angeordnet, dass mindestens ein Anteil einer transmittierten elektromagnetischen Strahlung 2.1 mindestens teilweise auf den Permittivitätssensor 5.1 fällt. Die transmittierte elektromagnetische Strahlung 2.1 ist derjenige Anteil der elektromagnetischen Strahlung 2, der den im Messraum 9 angeordneten Behälter 10 durchdringt.

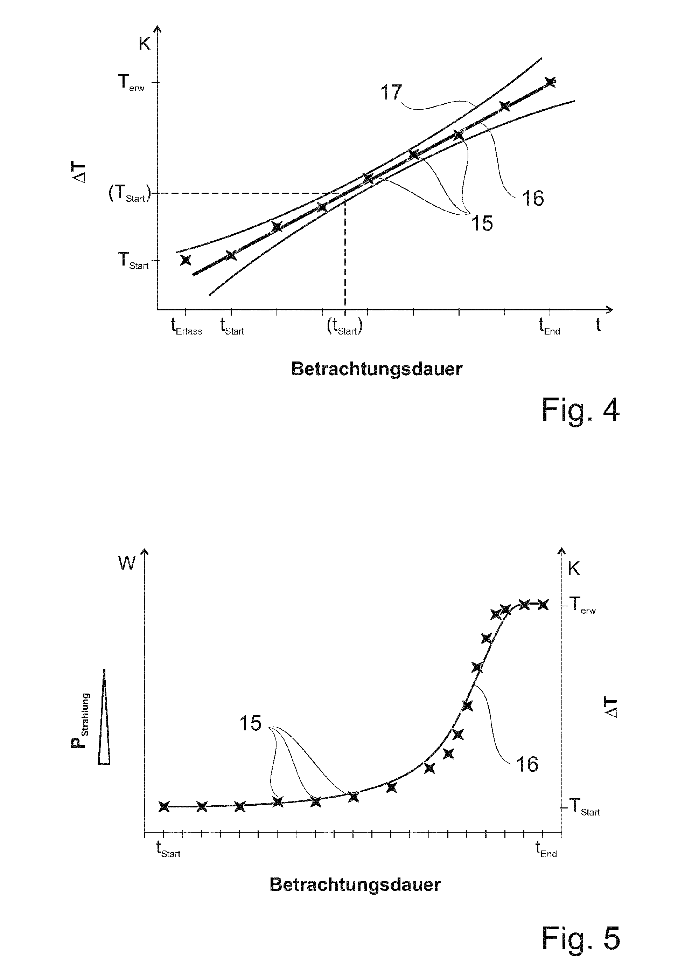

Die Strahlungsquelle 1 und der Permittivitätssensor 5.1 stehen signaltechnisch mit der Regel- und Steuereinheit 3 in Verbindung. Die Regel- und Steuereinheit 3 steht wiederum mit der Datenbank 4 in Verbindung. Die Datenbank 4 ist eine Rechner- und Speichereinheit, in der Daten abgelegt, gespeichert und wieder abgerufen werden können. Die Verbindung zwischen Regel- und Steuereinheit 3 und Datenbank 4 ist durch eine Kabelverbindung als Datenleitung realisiert. In weiteren Ausgestaltungen der Vorrichtung kann die Datenleitung auch drahtlos, beispielsweise per Funk oder optisch, umgesetzt sein. Die Regel- und Steuereinheit 3 weist eine Extrapolationseinheit 18 zur Extrapolation von Referenzmessreihen 16 und Bestrahlungsregimen sowie eine Überprüfungseinheit 19 zur Überprüfung der Übereinstimmung von Messwerten 15 mit Vertrauensbereichen 17 auf (siehe Fig. 4 bis 7).

Der Behälter 10 besteht aus einem für die elektromagnetische Strahlung 2 transmissiven Material. Er hat eine Behälterwand 10.1, durch die ein Innenraum 10.2 des Behälters 10 umschlossen ist. Über eine Zuleitung 12 kann das Medium 11 in den Innenraum 10.2 hinein- und über eine Ableitung 13 wieder aus dem Innenraum 10.2 herausgeleitet werden. Zuleitung 12 und Ableitung 13 weisen jeweils einen Verschluss 14 auf, durch dessen Wirkung die Zuleitung 12 bzw. die Ableitung 13 verschließbar sind. In einer weiteren Ausführung der Vorrichtung können die Verschlüsse 14 mit der Regel- und Steuereinheit 3 in Verbindung stehen und über diese mittels einer Verschlusseinheit (nicht gezeigt) geöffnet bzw. geschlossen werden.

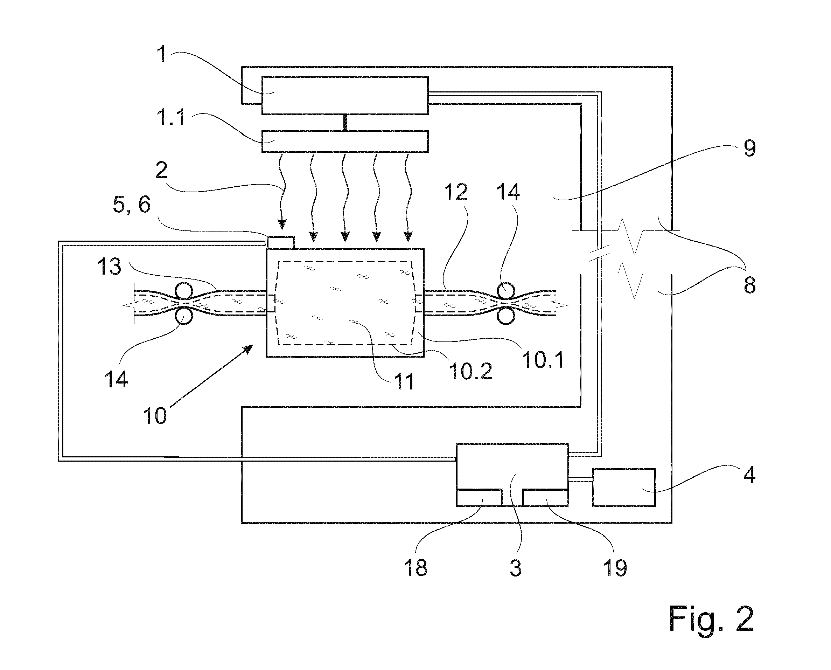

In Fig. 2 ist ein zweites Ausführungsbeispiel der erfindungsgemäßen Vorrichtung gezeigt, bei dem anstatt des Permittivitätssensors 5.1 ein Sensor 5 angeordnet ist, der zur Erfassung von Temperaturen des Mediums 11 im Innenraum 10.2 des Behälters 10 ausgelegt ist (fortan: Temperatursensor 6). Der Temperatursensor 6 ist außen an der Behälterwand 10.1 angeordnet und mit der Regel- und Steuereinheit 3 verbunden. In weiteren Ausführungen kann der Temperatursensor 6 auch im Innenraum 10.2 angeordnet sein. Ebenso sind Kombinationen der Anordnung der Sensoren 5 möglich.

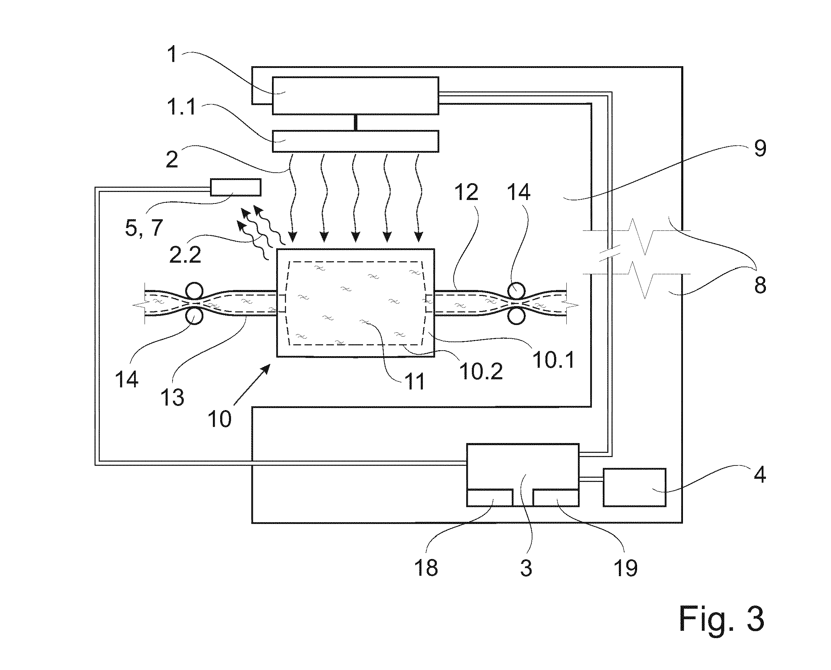

In einem dritten Ausführungsbeispiel gemäß Fig. 3 ist statt des Permittivitätssensors 5.1 oder des Temperatursensors 6 ein Sensor 5 angeordnet, der zur Erfassung einer reflektierten elektromagnetischen Strahlung 2.2 ausgelegt ist und als Reflektivitätssensor 7 bezeichnet wird. Die reflektierte elektromagnetische Strahlung 2.2 ist derjenige Anteil der in den Innenraum 10.2 abgestrahlten elektromagnetischen Strahlung 2, der von dem Material und der Oberfläche des Behälters 10 reflektiert wird. Reflektionen können an Grenzschichten des Materials sowie an Strukturen in dem Material auftreten.

Anhand der Fig. 4 soll der Zusammenhang zwischen Referenzmessreihen 16 und Bestrahlungsregime an einem vereinfachten Beispiel einer Vorrichtung mit Temperatursensor 6 (siehe auch Fig. 2) erläutert werden.

Um eine Referenzmessreihe 16 zu erhalten, wird vor der Durchführung der Erwärmung des Mediums 11 ein Referenzbehälter 10 in den Messraum 9 eingelegt. Der Referenzbehälter 10 entspricht hinsichtlich seiner inneren und äußeren Abmaße sowie des Materials einem erwartungsgemäß in der Praxis verwendeten Behälter 10 du ist daher mit dem gleichen Bezugszeichen versehen. In dem Referenzbehälter 10 ist ein Medium 11 vorhanden, das dem erwartungsgemäß in der Praxis verwendeten so weit als möglich entspricht oder diesem gleich ist.

Mittels der Strahlungsquelle 1 wird eine elektromagnetische Strahlung 2 erzeugt und durch die Abstrahleinrichtung 1.1 in den Innenraum 10.2 und auf den Referenzbehälter 10 abgestrahlt. Die Eigenschaften der elektromagnetischen Strahlung 2, wie Frequenz, Phasenlage und Leistung, sind bekannt. Zu Beginn der Erwärmung, also zum Startzeitpunkt t_{Start}, wird die Temperatur der Außenwand des Referenzbehälters 10 mittels des Temperatursensors 6 als ein erster Messwert 15 erfasst. Zur Erstellung der Referenzmessreihen 16 wird zudem durch einen hier nicht gezeigten Sensor die Temperatur des Mediums 11 erfasst und einer Außentemperatur der Behälterwand 10.1 des Referenzbehälters 10 zugeordnet gespeichert. Es wird so eine Korrelation zwischen den Temperaturen der Behälterwand 10.1 und dem Medium 11 erfasst. Damit ist es vorteilhaft ermöglicht, während der Verfahrensdurchführung allein aus der Temperatur der Behälterwand 10.1 auf die Temperatur des Mediums 11 zu schließen. Zu regelmäßigen Messzeitpunkten werden nun über die Bestrahlungsdauer die Temperaturen als jeweilige Messwerte 15 erfasst und den Messzeitpunkten zugeordnet abgespeichert. Die Erwärmung wird bei Erreichen eines Temperaturendpunktes T_{erw (erwünscht)} beendet, indem die Strahlungsquelle 1 durch die Regel- und Steuereinheit 3 angesteuert und abgeschalten wird. Durch die elektromagnetische Strahlung 2 ist eine Energie in das Medium 11 eingebracht worden, die zur Erwärmung des Mediums 11 auf einen Temperaturwert des Temperaturendpunktes T_{erw} führt.

In weiteren Ausführungen des erfindungsgemäßen Verfahrens kann die Strahlungsquelle 1 so angesteuert werden, dass die durch die elektromagnetische Strahlung 2 übertragene Energie reduziert wird und die Temperatur des Temperaturendpunktes T_{erw} in dem Medium 11 über eine bestimmte Zeitdauer beibehalten wird.

Die für die Erwärmung des Mediums 11 mittels der elektromagnetischen Strahlung 2 mit den bekannten Eigenschaften erforderliche Bestrahlungsdauer wird ermittelt und der Referenzmessreihe 16 zugeordnet abgespeichert. Die Referenzmessreihe 16 ist durch die Messwerte 15 gegeben. Anhand der Messwerte 15 ist eine Funktion der Referenzmessreihe 16 (als durchgehende Linie gezeigt) abgeleitet. Zu dieser Funktion wird ein Vertrauensbereich 17 berechnet und abgespeichert, wobei die Art und Weise der Berechnung des Vertrauensbereiches 17 vorab festgelegt ist. Im Beispiel ist der Vertrauensbereich 17 unter Berücksichtigung der bekannten Messfehler des Temperatursensors 6 und der gerätetechnisch bedingten Toleranzen bei der Erzeugung, Bereitstellung und Abstrahlung der elektromagnetischen Strahlung 2 sowie unter Zugrundelegen einer geeigneten Wahrscheinlichkeitsverteilung (z. B. Gauss-, Weibull-, Poissonverteilung etc.) berechnet.

Im Beispiel ist im Zuge der Erwärmung des Mediums 11 der Temperaturendpunkt T_{erw} genau zum Ende der Bestrahlungsdauer erreicht. Es bietet sich also an, die Funktion der Referenzmessreihe 16 zugleich als Bestrahlungsregime zu verwenden, dieses der Referenzmessreihe 16 zuzuordnen und abzuspeichern.

Solche Referenzmessreihen 16, Vertrauensbereiche 17 und Bestrahlungsregime werden zum Aufbau der Datenbank 4 für eine Vielzahl von Referenzbehältern 10 aus unterschiedlichen Materialien und / oder unterschiedlichen Abmessungen sowie für verschiedene Medien 11 ermittelt und in der Datenbank 4 abgespeichert. Weiterhin werden die Eigenschaften der elektromagnetischen Strahlung 2 variiert. Diese können über die Bestrahlungsdauer konstant, linear verändert oder nicht-linear verändert sein. Jede Variation der elektromagnetischen Strahlung 2 wird als eine der ersten bis n-ten elektromagnetischen Strahlung 2 abgespeichert.

Es ist möglich, die Daten der Datenbank 4 für jede einzelne Vorrichtung zu ermitteln. Günstiger ist es jedoch, wenn bereits vorbekannte Daten in der Datenbank 4 abgelegt werden. Diese können durch spezifische Daten der jeweiligen Vorrichtung ergänzt und / oder korrigiert werden.

Bei der Durchführung des erfindungsgemäßen Verfahrens ist statt des Referenzbehälters 10 ein Behälter 10 mit einem Medium 11 in dem Messraum 9 angeordnet. Als erstes wird die Temperatur des Mediums 11 erfasst und als Starttemperatur T_{Start} an die Regel- und Steuereinheit 3 weitergeleitet. Es erfolgt ein Abgleich der erfassten Starttemperatur T_{Start} mit in der Datenbank 4 abgelegten Starttemperaturen T_{Start}. Diejenigen Referenzmessreihen 16, die eine gleiche Starttemperatur T_{Start} aufweisen, werden vorausgewählt. Dabei werden auch die Referenzmessreihen 16 vorausgewählt, die einen Temperaturwert gleich der erfassten Starttemperatur T_{Start} in ihrem Vertrauensbereich 17 beinhalten. Die Referenzmessreihe 16, deren Funktion den Wert der erfassten Starttemperatur T_{Start} beinhaltet, soll im hier gegebenen Beispiel ausgewählt werden. Das zur ausgewählten Referenzmessreihe 16 zugeordnete Bestrahlungsregime wird ebenfalls ausgewählt. Entsprechend diesem ausgewählten Bestrahlungsregime wird die Strahlungsquelle 1 durch die Regel- und Steuereinheit 3 zur Erwärmung des Mediums 11 angesteuert. Da die derzeit verwendeten Regel- und Steuereinheiten 3 sowie die Datenbanken 4 sehr kurze Zeiten für die beschriebene Temperaturerfassung, Auswahl der Referenzmessreihe 16 und Ansteuerung der Strahlungsquelle 1 erlauben, können diese Schritte zu Beginn der Bestrahlungsdauer durchgeführt werden.

Es ist in weiteren Ausgestaltungen möglich, dass die Temperatur des Mediums 11 zu einem Erfassungszeitpunkt t_{Erfass} erfolgt, der vor dem Startzeitpunkt t_{Start} liegt. Die oben beschriebenen Schritte der Temperaturerfassung und der Auswahl der Referenzmessreihe 16 werden vor dem Startzeitpunkt t_{Start} ausgeführt. Die Erwärmung beginnt zum Startzeitpunkt t_{Start} mit der Ansteuerung der Strahlungsquelle 1 durch die Regel- und Steuereinheit 3.

Mit Beginn der Erwärmung wird durch die Regel- und Steuereinheit 3 die Strahlungsquelle 1 entsprechend dem ausgewählten Bestrahlungsregime angesteuert. Die elektromagnetische Strahlung 2 wird erzeugt, bereitgestellt und über die Abstrahleinrichtung 1.1 in den Messraum 9 abgestrahlt. Die Erwärmung des Mediums 11 wird entsprechend dem ausgewählten Bestrahlungsregime durchgeführt.

In einer weiteren Ausgestaltung des Verfahrens wird zu bekannten Messzeitpunkten innerhalb der Bestrahlungsdauer die Temperatur des Mediums 11 dem Messzeitpunkt zugeordnet als weitere Messwerte 15 erfasst und der jeweilige Messwert 15 an die Regel- und Steuereinheit 3 geleitet. Dort wird überprüft, ob der Messwert 15 zu dem entsprechenden (Mess-)Zeitpunkt innerhalb des Vertrauensbereiches 17 der Referenzmessreihe 16 liegt. Ist dies der Fall, wird die Erwärmung mit dem Bestrahlungsregime fortgesetzt.

Ergibt die Überprüfung, dass der weitere Messwert 15 nicht innerhalb des Vertrauensbereiches 17 liegt, wird ein Fehlersignal erzeugt. In einer Ausgestaltung ist vorgesehen zu überprüfen, ob mindestens der auf den abweichenden weiteren Messwert 15 folgende weitere Messwert 15 wieder innerhalb des Vertrauensbereiches 17 liegt. In diesem Fall wird die Erwärmung mit dem ausgewählten Bestrahlungsregime fortgesetzt, bis die Bestrahlungsdauer beendet ist oder der Temperaturendpunkt t_{erw} erreicht ist, je nachdem, welches Kriterium für die Beendigung der Erwärmung gewählt wurde.

Weicht mindestens ein weiterer Messwert 15 von dem Vertrauensbereich 17 ab, wird in einer weiteren Ausgestaltung des Verfahrens durch die Regel- und Steuereinheit 3 die Datenbank 4 durchsucht, um eine andere Referenzmessreihe 16 sowie ein zugeordnetes Bestrahlungsregime zu finden, mit dem die Erwärmung fortgesetzt werden kann. Dabei erfolgt die Auswahl der anderen Referenzmessreihe 16 anhand mindestens eines weiteren Messwerts 15, in verbesserten Ausgestaltungen aber anhand mehrerer weiterer Messwerte 15. Die auf diese Weise aus der ausgewählten Referenzmessreihe 16 und der anderen Referenzmessreihe 16 zusammengesetzte Referenzmessreihe 16 wird in einer weiterführenden Ausgestaltung des Verfahrens in der Datenbank 4 abrufbar gespeichert.

Entsprechend der vorgenannten Vorgehensweise werden auch diejenigen Ausgestaltungen des Verfahrens durchgeführt, bei denen eine andere Messgröße und / oder eine abgeleitete Messgröße verwendet werden.

In weiteren Ausgestaltungen des erfindungsgemäßen Verfahrens können auch mehrere Messgrößen und / oder abgeleitete Messgrößen verwendet werden (Temperatur, Reflektivität, Transmissivität, Realteil der Permittivität und / oder Imaginärteil der Permittivität). Durch die Verwendung mehrerer Messgrößen und / oder abgeleiteter Messgrößen beruht die Auswahl der Referenzmessreihe 16 und des zugeordneten Bestrahlungsregimes auf Wertetripeln, Wertequadrupeln, usw., wodurch eine verbesserte Sicherheit dafür erlangt wird, dass das Bestrahlungsregime der ausgewählten Referenzmessreihe 16 auch über die gesamte Bestrahlungsdauer beibehalten werden kann. Alternativ kann eine Auswahl anhand einer Messgröße oder abgeleiteten Messgröße getroffen und durch die Auswahl anhand mindestens einer weiteren Messgröße oder abgeleiteten Messgröße überprüft werden.

In einer alternativen Verfahrensführung der Schritte A bis D kann die Erwärmung auch mit Ablauf der Bestrahlungsdauer beendet werden. In diesem Fall wird anschließend überprüft, ob der Temperaturendpunkt T_{erw} innerhalb der Bestrahlungsdauer erreicht wurde und zu welchem Zeitpunkt innerhalb der Bestrahlungsdauer dies gegebenenfalls geschehen ist. Wird der Temperaturendpunkt T_{erw} innerhalb der Bestrahlungsdauer erreicht, wird die Referenzmessreihe 16 ausgewählt. Um ein Überschreiten des Temperaturendpunktes T_{erw} zu vermeiden, wird ein der Referenzmessreihe 16 zugeordnetes Bestrahlungsregime so gestaltet, dass die Leistung der abgestrahlten elektromagnetischen Strahlung 2 (abgestrahlte Leistung) reduziert wird.

Wird der Temperaturendpunkt T_{erw} innerhalb der Bestrahlungsdauer nicht erreicht, wird die Referenzmessreihe 16 verworfen. Alternativ kann die Referenzmessreihe 16 als Grundlage zur Ermittlung (z. B. durch Extrapolation) eines extrapolierten Bestrahlungsregimes genutzt werden.

Ein weiteres Beispiel eines Bestrahlungsregimes und einer Referenzmessreihe 16 ist in Fig. 5 dargestellt. Die Messwerte 15 der Referenzmessreihe 16 sind durch Kreuze angegeben. Die Messwerte 15 der Messgröße Temperatur wurden über die Bestrahlungsdauer (zwischen dem Startzeitpunkt t_{Start} und dem Endzeitpunkt t_{End}) mittels eines Temperatursensors 6 erfasst. Zugleich wurde die Leistung der abgestrahlten elektromagnetischen Strahlung 2 über die Bestrahlungsdauer in nichtlinearer Weise verringert (durchgehende Linie). Es ist zu erkennen, dass die Temperatur des Mediums 11 trotz hoher Leistung der elektromagnetischen Strahlung 2 anfänglich nur langsam ansteigt. Etwa zu Beginn der zweiten Hälfte der Bestrahlungsdauer steigt die Temperatur exponentiell mit der Zeit an, obwohl die Leistung der elektromagnetischen Strahlung 2 reduziert wird. Dies kann beispielsweise auf temperaturabhängige Energieaufnahmen (Absorption) des Mediums 11 und / oder des Materials des Behälters 10 sowie aufgrund von Effekten wie konstruktiven oder destruktiven Interferenzen der elektromagnetischen Strahlung 2 zurückzuführen sein. Um ein Überschreiten des Temperaturendpunktes T_{erw} zu vermeiden, wird gegen Ende der Bestrahlungsdauer die Leistung je Zeiteinheit immer stärker verringert. Der gezeigten Referenzmessreihe 16 (Temperatur über der Zeit) ist das gezeigte Bestrahlungsregime (Leistung der Strahlung über der Zeit) zugeordnet und beide sind in der Datenbank 4 abgelegt.

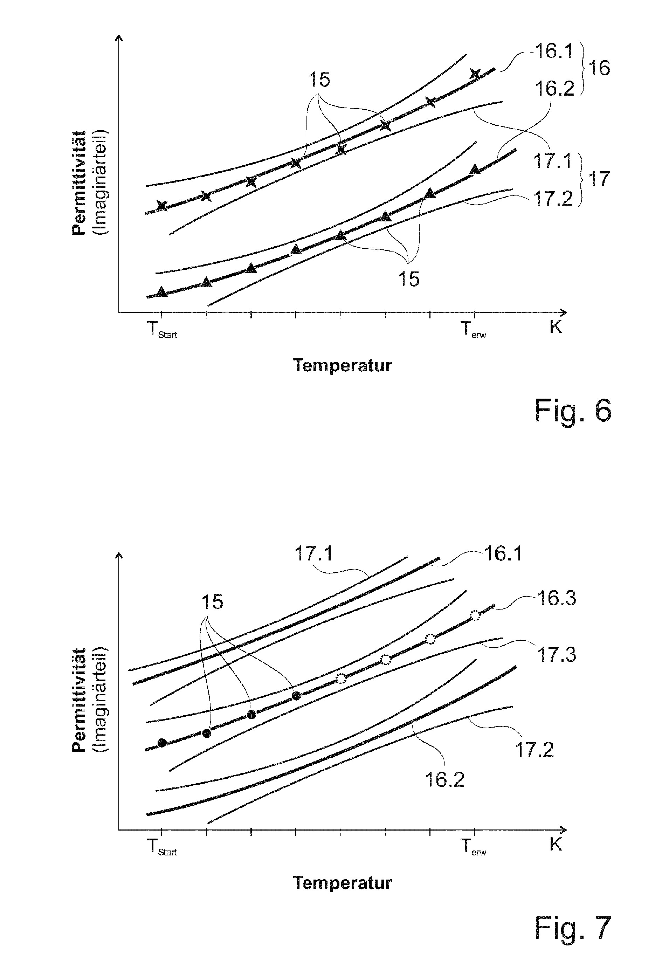

In einem in Fig. 6 gezeigten Diagramm sind beispielhaft zwei Referenzmessreihen 16 mit Vertrauensbereichen 17 gezeigt. Auf der Abszisse sind Temperaturen in Grad Celsius aufgetragen. Eine Starttemperatur T_{Start}, von der aus eine Erwärmung des Mediums 11 in dem Behälter 10 beginnt, und ein Temperaturendpunkt T_{erw}, bei dem eine erwünschte Temperatur des Mediums 11 erreicht ist, sind gekennzeichnet. Auf der Ordinate ist der Imaginärteil der erfassten Permittivität der elektromagnetischen Strahlung 2 bei Durchtritt durch den mit Medium 11 gefüllten Behälter 10 aufgetragen. Der Imaginärteil der erfassten Permittivität ist eine abgeleitete Messgröße.

Eine erste Referenzmessreihe 16.1 ist durch eine Funktion dargestellt, die aus einer Regression der Messwerte 15 (durch Kreuze symbolisiert) der ersten Referenzmessreihe 16.1 abgeleitet wurde. Anhand der Messwerte 15 und der Funktion wurde ein erster Vertrauensbereich 17.1 der Funktion als 95%-Vertrauensbereich berechnet. Die erste Referenzmessreihe 16.1 ist durch eine Bestrahlung eines Mediums 11 in dem Behälter 10 mittels einer elektromagnetischen Strahlung 2 bekannter Wellenlänge_und Leistung ermittelt worden. Während der Erwärmung des Mediums von der Starttemperatur T_{Start} auf den Temperaturendpunkt T_{erw} ist die Permittivität als Messgröße erfasst und deren jeweiliger Imaginärteil als abgeleitete Messgröße ermittelt worden. Es wird hier angenommen, dass die Erwärmung des Mediums von der Starttemperatur T_{Start} auf den Temperaturendpunkt T_{erw} innerhalb einer vorgegebenen Bestrahlungsdauer von 15 Sekunden erfolgte.

Gleiches gilt für eine zweite Referenzmessreihe 16.2. Durch Regression der Messwerte 15 (durch Dreiecke symbolisiert) der zweiten Referenzmessreihe 16.2 sind eine Funktion und ein zweiter Vertrauensbereich 17.2 der Funktion als 95%-Vertrauensbereich berechnet.

Erster und zweiter Vertrauensbereich 17.1, 17.2 überschneiden sich über den dargestellten Funktionsbereich nicht.

Die erste Referenzmessreihe 16.1 ist durch Messwerte 15 erhalten worden, die bei einer bestimmten Wellenlänge und Leistung der abgestrahlten elektromagnetischen Strahlung 2 erfasst wurden. Der zeitliche Ablauf der Leistungseinbringung in das Medium 11 mittels der elektromagnetischen Strahlung 2 stellt ein Bestrahlungsregime dar. In einem einfachen Fall wurden die Eigenschaften der elektromagnetischen Strahlung 2 über die Bestrahlungsdauer nicht verändert. Wie der Verlauf der ersten Referenzmessreihe 16.1 zeigt, stieg aber der Imaginärteil der Permittivität nichtlinear mit der Temperatur an.

Eine weitere Ausgestaltung des erfindungsgemäßen Verfahrens ist in Fig. 7 veranschaulicht. Zu sehen sind eine erste Referenzmessreihe 16.1 mit einem ersten Vertrauensbereich 17.1 und eine zweite Referenzmessreihe 16.2 mit einem zweiten Vertrauensbereich 17.2. Zwischen den beiden Referenzmessreihen 16.1, 16.2 ist eine dritte Referenzmessreihe 16.3 zu sehen, die nur aus wenigen (gezeigt sind vier) Messwerten 15 besteht. Aufgrund der Kenntnis der in der Datenbank 4 abgelegten Referenzmessreihen 16, deren Funktionen und Vertrauensbereichen 17 sowie der Bestrahlungsregime ist es möglich, den Verlauf der dritten Referenzmessreihe 16.3 bis zum Erreichen des Temperaturendpunktes T_{erw} ausgehend von den Messwerten 15 zu extrapolieren. Der weitere Verlauf der dritten Referenzmessreihe 16.3, d. h. weitere, imaginäre Messwerte 15 (gestrichelt umrandet dargestellt), wird also anhand bekannter Daten geschätzt. Für die so extrapolierte dritte Referenzmessreihe 16.3 kann anschließend in bekannter Weise ein dritter Vertrauensbereich 17.3 berechnet werden. Anhand der dritten Referenzmessreihe 16.3 kann außerdem ein geeignetes Bestrahlungsregime prognostiziert werden. Das so prognostizierte Bestrahlungsregime kann bei seiner Anwendung mittels einer Erfassung tatsächlicher Messwerte 15 überprüft und gegebenenfalls korrigiert werden.

### Bezugszeichenliste

- 1: Strahlungsquelle
- 1.1: Abstrahleinrichtung
- 2: elektromagnetische Strahlung
- 2.1: transmittierte elektromagnetische Strahlung
- 2.2: reflektierte elektromagnetische Strahlung
- 3: Regel- und Steuereinheit
- 4: Datenbank
- 5: Sensor
- 5.1: Permittivitätssensor
- 6: Temperatursensor
- 7: Reflektivitätssensor
- 8: Rahmen
- 9: Messraum
- 10: Behälter, Referenzbehälter
- 10.1: Behälterwand
- 10.2: Innenraum
- 11: Medium
- 12: Zuleitung
- 13: Ableitung
- 14: Verschluss
- 15: Messwerte
- 16: Referenzmessreihe
- 16.1: erste Referenzmessreihe
- 16.2: zweite Referenzmessreihe
- 16.3: dritte Referenzmessreihe
- 17: Vertrauensbereich
- 17.1: erster Vertrauensbereich
- 17.2: zweiter Vertrauensbereich
- 17.3: dritter Vertrauensbereich
- 18: Extrapolationseinheit
- 19: Überprüfungseinheit

## Patentansprüche

1. Verfahren zur Erwärmung von Volumina von Medien in einem geschlossenen Behälter mittels einer elektromagnetischen Strahlung, wobei vor der Durchführung der Erwärmung eine Datenbank (4) angelegt wird, indem
in einem Schritt A mindestens ein Referenzbehälter (10) aus einem bekannten Material, der ein bekanntes Volumen aufweist und ein bekanntes Medium (11) enthält, mit jeweils einer ersten bis n-ten elektromagnetischen Strahlung (2) über je eine Zeitdauer bestrahlt wird, wobei die Eigenschaften der ersten bis n-ten elektromagnetischen Strahlung (2) bekannt sind,
in einem Schritt B über die Zeitdauer der Bestrahlung zu bekannten Messzeitpunkten eine Anzahl Messwerte (15) mindestens einer Messgröße erhoben und als dem Referenzbehälter (10) zugeordnete Messreihen erfasst und abrufbar als Referenzmessreihen (16) abgespeichert werden,
in einem Schritt C aus den Referenzmessreihen (16) diejenigen Referenzmessreihen (16) ausgewählt werden, bei deren Erstellung innerhalb einer ausgewählten Bestrahlungsdauer eine ausgewählte Energiemenge in den Referenzbehälter (10) eingekoppelt worden ist,
in einem Schritt D von den ausgewählten Referenzmessreihen (16) jeweils ein Bestrahlungsregime abgeleitet wird und diesen Referenzmessreihen (16) zugeordnet abgespeichert wird,
und bei dem bei der Durchführung der Erwärmung
in einem Schritt a der Behälter (10) mit der elektromagnetischen Strahlung (2), deren Eigenschaften bekannt sind, über eine bekannte Bestrahlungsdauer bestrahlt wird und mindestens ein dem Behälter (10) und einem Messzeitpunkt innerhalb der Bestrahlungsdauer zugeordneter Messwert (15) einer Messgröße erhoben und auf das Vorliegen einer Übereinstimmung mit den Werten von in einer Datenbank abgelegten Referenzmessreihen (16) überprüft wird, danach
in einem Schritt b bei einer Übereinstimmung des mindestens einen Messwertes (15) mit einem Wert einer Referenzmessreihe (16) ein der betreffenden Referenzmessreihe (16) zugeordnetes Bestrahlungsregime ausgewählt wird, und dann
in einem Schritt c die Erwärmung des Behälters (10) unter Anwendung des ausgewählten Bestrahlungsregimes bis zu einem vorbestimmten Temperaturendpunkt T_{erw} fortgesetzt wird.

2. Verfahren nach Anspruch 1, **dadurch gekennzeichnet, dass** während der Erwärmung weitere Messwerte (15) erhoben werden und eine Übereinstimmung mindestens einer Auswahl der weiteren Messwerte (15) mit Werten derjenigen Referenzmessreihe (16), der das ausgewählte Bestrahlungsregime zugeordnet ist, überprüft wird.

3. Verfahren nach Anspruch 2, **dadurch gekennzeichnet, dass** bei einer fehlenden Übereinstimmung ein anderes Bestrahlungsregime ausgewählt und die Erwärmung mit dem ausgewählten anderen Bestrahlungsregime fortgesetzt wird.

4. Verfahren nach Anspruch 3, **dadurch gekennzeichnet, dass** die Erwärmung beendet und ein Fehlersignal generiert wird, wenn kein anderes Bestrahlungsregime auswählbar ist.

5. Verfahren nach Anspruch 2, **dadurch gekennzeichnet, dass** bei einer fehlenden Übereinstimmung mindestens eines weiteren Messwertes (15) aufgrund der bis dahin erfassten weiteren Messwerte (15) ein prognostiziertes Bestrahlungsregime berechnet wird und die Erwärmung mit dem prognostizierten Bestrahlungsregime fortgesetzt wird.

6. Verfahren nach Anspruch 5, **dadurch gekennzeichnet, dass** das prognostizierte Bestrahlungsregime in die Datenbank (4) übernommen und wiederholt abrufbar gespeichert wird.

7. Verfahren nach Anspruch 1, **dadurch gekennzeichnet, dass** die eine Messgröße aus einer Reflektivität des Behälters (10), einer Transmissivität des Behälters (10), mindestens einer Temperatur an mindestens einem ausgewählten Punkt des Behälters (10) oder einer frequenzabhängigen Phasenverschiebung eines den Behälter (10) durchdringenden Anteils der elektromagnetischen Strahlung (2) ausgewählt wird.

8. Verfahren nach Anspruch 1, **dadurch gekennzeichnet, dass** mindestens zwei Messgrößen aus einer Gruppe von Messgrößen, bestehend aus einer Reflektivität, einer Transmissivität, einer Temperatur und einer Phasenverschiebung der elektromagnetischen Strahlung (2), ausgewählt werden.

9. Vorrichtung zur Erwärmung der Volumina von Medien in einem geschlossenen Behälter mittels einer elektromagnetischen Strahlung aufweisend
- eine Strahlungsquelle zur Bereitstellung einer elektromagnetischen Strahlung,
- einen Messraum zur Aufnahme des Behälters und mit der Strahlungsquelle und eine Abstrahleinrichtung zur gerichteten Abstrahlung der elektromagnetischen Strahlung in den Messraum,
- eine Regel- und Steuereinheit zur Regelung und Steuerung der Bereitstellung und der Abstrahlung der elektromagnetischen Strahlung entsprechend eines Bestrahlungsregimes,
**dadurch gekennzeichnet, dass**
- die Regel- und Steuereinheit (3) mit mindestens einem Sensor (5, 5.1, 6, 7) in Verbindung steht und der mindestens eine Sensor (5, 5.1, 6, 7) zur Erfassung mindestens eines dem Behälter (10) zugeordneten Messwerts (15) einer Messgröße ausgebildet ist,
- die Regel- und Steuereinheit (3) datentechnisch mit der Datenbank (4) verbunden ist, in der eine Anzahl von Referenzmessreihen (16) sowie eine Anzahl von den jeweiligen Referenzmessreihen (16) zugeordneten Bestrahlungsregimen abgelegt sind,
- die Regel- und Steuereinheit (3) eine Überprüfungseinheit (19) zur Überprüfung einer Übereinstimmung des mindestens einen erfassten Messwerts (15) mit einem Wert der Referenzmessreihen (16) und, bei festgestellter Übereinstimmung, zur Zuordnung eines Bestrahlungsregimes zu dem erfassten und überprüften Messwert (15) aufweist.

10. Vorrichtung nach Anspruch 9, **dadurch gekennzeichnet, dass** die Regel- und Steuereinheit (3) eine Extrapolationseinheit (18) zur Ableitung von Bestrahlungsregimen aus einer Anzahl von Messwerten (15) aufweist.

11. Vorrichtung nach einem der Ansprüche 9 oder 10, **dadurch gekennzeichnet, dass** der Sensor (5) ein Reflektivitätssensor (7) zur Erfassung mindestens einer Reflektivität des Behälters (10) ist.

12. Vorrichtung nach einem der Ansprüche 9 bis 11, **dadurch gekennzeichnet, dass** der Sensor (5) ein Permittivitätssensor (5.1) zur Erfassung mindestens einer frequenzabhängigen Phasenverschiebung eines den Behälter (10) durchdringenden Anteils einer transmittierten elektromagnetischen Strahlung (2.1) ist.

13. Vorrichtung nach einem der Ansprüche 9 bis 12, **dadurch gekennzeichnet, dass** der Sensor (5) ein Temperatursensor (6) zur Erfassung einer Temperatur des Behälters (10) ist.

14. Vorrichtung nach einem der Ansprüche 9 bis 13, **dadurch gekennzeichnet, dass** der Sensor (5) zur Erfassung einer Transmissivität ausgebildet ist.

15. Verwendung des Verfahrens nach den Ansprüchen 1 bis 8 zur Desinfektion des Inneren des Behälters (10), wobei der Behälter (10) ein medizinisches Gerät oder ein Bestandteil eines medizinischen Geräts ist.

16. Verwendung nach Anspruch 15, **dadurch gekennzeichnet, dass** der Behälter (10) ein Luer®-Verbinder (Luer®-Lock) ist.
